Europäisches Patentamt

European Patent Office

Office européen des brevets

(11) Numéro de publication: **0 317 378 B2**

# (12) NOUVEAU FASCICULE DE BREVET EUROPEEN

(45) Date de publication de nouveau fascicule du brevet: 30.08.95

(51) Int. Cl.⁶: $C01B$ 33/193, $A61K$ 7/16

(21) Numéro de dépôt: 88402653.5

(22) Date de dépôt: 21.10.88

(54) **Silice pour compositions dentifrices compatible notamment avec le zinc.**

(30) Priorité: 04.11.87 FR 8715276

(43) Date de publication de la demande:
24.05.89 Bulletin 89/21

(45) Mention de la délivrance du brevet:
25.03.92 Bulletin 92/13

(45) Mention de la décision
concernant l'opposition:
30.08.95 Bulletin 95/35

(84) Etats contractants désignés:
AT BE CH DE ES FR GB GR IT LI LU NL SE

(56) Documents cités:
EP-B- 0 031 271      EP-B- 0 078 909
DE-A- 2 920 906      GB-A- 1 580 672
US-A- 3 070 426      US-A- 4 272 509

Patent Abstracts of Japan, vol. 10, no. 67(C-333)(2124), 15 Mai 1986; & JP-A-60204613 (Nihon Ita Glass K.K.) 16-10-1985

Patent Abstracts of Japan, vol. 11, no. 251 (C-440)(2698), 14 Aout 1987); & JP-A-6256319 (Nippon Chem. Ind. Co. Ltd.) 12-03-1987

(73) Titulaire: **RHONE-POULENC CHIMIE**
**25, Ouai Paul Doumer**
**F-92408 Courbevoie Cédex (FR)**

(72) Inventeur: **Persello, Jacques**
**14 Chemin de Calice**
**310 - La Boisse**
**F-01120 Montluel (FR)**

(74) Mandataire: **Dubruc, Philippe et al**
**RHONE-POULENC CHIMIE,**
**Direction de la Propriété Industrielle,**
**25, Ouai Paul Doumer**
**F-92408 Courbevoie Cédex (FR)**

**Description**

La présente invention concerne une silice utilisable notamment dans les compositions dentifrices, ainsi que des compositions dentifrices comprenant cette silice.

On sait que la silice est couramment utilisée dans la préparation de compositions dentifrices. Elle peut y jouer d'ailleurs plusieurs rôles.

Elle agit tout d'abord comme agent abrasif en aidant par son action mécanique à l'élimination de la plaque dentaire.

Elle peut aussi jouer le rôle d'agent épaississant pour conférer des propriétés rhéologiques déterminées au dentifrice ainsi que d'agent optique pour lui donner la coloration souhaitée.

Par ailleurs, on sait que les dentifrices contiennent des agents divers notamment pour la prévention des caries, pour diminuer la formation de la plaque dentaire ou le dépôt de tartre sur les dents. Parmi ces agents on peut citer en particulier le zinc. D'autres éléments sont aussi utilisés tels les fluorures phosphates, les pyrophosphates, les polyphosphates, les polyphosphonates. Les formulations dentifrices peuvent aussi comporter des aromes, des parfums, etc...

La présence de ces agents dans le dentifrice pose le problème de leur compatibilité avec la silice. En effet à cause notamment de ses capacités absorbantes, celle-ci peut avoir tendance à réagir avec ces agents de telle sorte qu'ils ne soient plus disponibles pour exercer les effets thérapeutiques décrits plus haut.

L'objet de l'invention est donc de trouver des silices compatibles avec les agents mentionnés ci-dessus notamment avec le zinc et donc parfaitement utilisables dans la formulation de dentifrices.

Or, la Demanderesse s'est aperçu que les propriétés de compatibilité recherchées dépendaient essentiellement de la chimie de surface de la silice utilisée. On a pu ainsi établir un certain nombre de conditions sur la surface des silices pour que celles-ci soient compatibles.

Dans ce but, la silice de l'invention, utilisable notamment dans les compositions dentifrices est caractérisée en ce qu'elle présente une chimie de surface telle que le nombre de OH exprimé en OH/nm2 soit d'au plus 15 et que son point de charge nulle (PZC) soit compris entre 3 et 6,5 et une compatibilité avec le zinc d'au moins 50 %.

Le procédé de préparation de la silice de l'invention est du type comprenant une réaction d'un silicate avec un acide ce par quoi on obtient une suspension ou un gel de silice, une séparation et un séchage de la silice et il est caractérisé en ce qu'après la séparation de la silice de la suspension, on effectue un premier lavage à l'eau du gâteau résultant puis un second lavage ou un traitement avec une solution acide.

L'invention concerne aussi des compositions dentifrices caractérisées en ce qu'elles contiennent des silices telles que décrites ci-dessus.

D'autres caractéristiques et avantages de l'invention seront mieux compris à la lecture de la description et des exemples concrets qui vont suivre.

Comme cela a été indiqué, en introduction, les caractéristiques essentielles des silices de l'invention résident dans leur chimie de surface. Plus précisément, un des aspects à prendre en compte dans cette chimie de surface est l'acidité. A ce sujet une des caractéristiques des silices de l'invention est le nombre des sites acides de surface.

Ce nombre se mesure en nombre de groupements OH ou silanols par nm2. En pratique, la mesure se fait de la manière suivante.

Le nombre de sites OH de surface est assimilé à la quantité d'eau libérée entre 190° c et 900°C.

Les échantillons de silice sont préalablement séchés à 105°C pendant 2 heures.

Une masse Po de silice est placée dans une thermobalance et portée à 190°C pendant 2 heures; soit P190 la masse obtenue. La silice est portée ensuite à 900°C pendant 2 heures, soit P900 la nouvelle masse obtenue.

Le nombre de sites OH est calculé par l'équation suivante :

$$NOH = \frac{66922,2}{A} \times (P190-P900)/190$$

où NOH est nombre de sites OH par nm2 de surface

A est la surface spécifique du solide (BET) en m2/g.

Dans le cas présent, les silices de l'invention présentent un nombre de OH/nm2 d'au plus 15. Selon un mode préféré de l'invention ce nombre de OH est d'au plus 12 et notamment compris entre 3 et 12.

D'autre part, le pH de surface est aussi une caractéristique des silices de l'invention. Il est déterminé par le point de charge nulle PZC.

Ce point de charge nulle (PZC) est défini par le pH d'une suspension de silice pour lequel la charge électrique de la surface du solide est nulle et ce quelle que soit la force ionique du milieu. Ce PZC mesure le pH réel de la surface, dans la mesure où celle-ci est libre de toutes impuretés de type ionique.

La charge électrique est déterminée par potentiométrie. Le principe de la méthode est basé sur le bilan global des protons absorbés ou désorbés sur la surface de la silice à un pH donné.

A partir des équations décrivant le bilan global de l'opération, il est facile de montrer que la charge électrique C de la surface, prise par rapport à une référence correspondant à une charge de surface nulle, est donnée par l'équation :

$$C = \frac{F}{A.M.} (H - OH)$$

dans laquelle :

A représente la surface spécifique du solide en $m^2/g$,

M est la quantité de solide dans la suspension en g,

F est le Faraday,

H ou OH représente la variation par unité de surface de l'excès d'ions $H^+$ ou $OH^-$ respectivement sur le solide.

La mesure expérimentale du PZC se fait de la manière suivante.

On utilise la méthode décrite par Berube et de Bruyn (J. Colloid Interface Sc. 1968, 27, 305).

La silice est lavée au préalable dans de l'eau déionisée de haute résistivité (10 Mega.Ohm.cm); séchée puis dégazée.

On prépare une série de solutions à pHo 8,5 par ajout de KOH ou HNO3 et contenant un électrolyte indifférent (KNO3) a une concentration variable entre $10^{-5}$ et $10^{-1}$ Mole/l.

A ces solutions on ajoute une masse donnée de silice et on laisse le pH des suspensions obtenues se stabiliser sous agitation, à 25°C et sous azote pendant 24 heures ; soit pH'o sa valeur.

Des solutions étalons sont constituées par le surnageant obtenu par centrifugation pendant 30 mn à 10000 t/mn d'une partie de ces mêmes suspensions. Soit pH'o le pH de ces surnageants.

On ramène ensuite le pH d'un volume connu de ces suspensions et des solutions étalons correspondantes à pHo en rajoutant la quantité nécessaire de KOH et on laisse les suspensions et les solutions étalons se stabiliser pendant 4 heures.

Le dosage potentiométrique des suspensions est effectué à partir de pHo par addition d'acide nitrique jusqu'à pHf = 2,0.

Préférentiellement on procède par addition d'incrément d'acide correspondant à une variation de pH de 0,2 unité de pH. Après chaque addition, le pH est stabilisé pendant 1 mn.

Soit Vh.Nh le nombre d'équivalents d'acide pour parvenir à pHf.

A partir de pHo, on trace le terme (Vh.Nh - Voh.Noh) en fonction des pH incrémentés pour toutes les suspensions (3 forces ioniques au moins) et pour toutes les solutions étalons correspondantes.

Pour chaque valeur de pH (pas de 0,2 unité) on fait ensuite la différence entre la consommation de $H^+$ ou $OH^-$ pour la suspension et pour la solution étalon correspondante. On renouvelle cette opération pour toutes les forces ioniques.

Ceci donne le terme (H - OH) correspondant à la consommation en protons de la surface. La charge de surface est calculée par l'équation ci-dessus.

On trace ensuite les courbes charge de surface en fonction du pH pour toutes les forces ioniques considérées. Le PZC est défini par l'intersection des courbes.

On ajuste la concentration en silice en fonction de la surface spécifque de celle-ci.

Par exemple, on utilise des suspensions à 2% pour les silices de 50 m2/g à 3 forces ioniques (0,1 ; 0,01 et 0,001 mole/l).

Le dosage est effectué sur 100 ml de suspension en utilisant de l'hydroxyde de potassium à 0,1 M.

Pour les silices de l'invention ce PZC doit être compris entre 3 et 6,5.

Par ailleurs pour améliorer la compatibilité des silices de l'invention vis-à-vis d'autres éléments que le zinc, notamment vis-à-vis du fluor, il est intéressant que leur teneur en aluminium soit au plus de 500 ppm.

D'autre part la teneur en fer des silices de l'invention peut être avantageusement d'au plus 200 ppm.

Par ailleurs, d'une manière préférentielle la teneur en calcium peut être d'au plus 500 ppm et plus particulièrement d'au plus 330 ppm.

Les silices de l'invention présentent aussi généralement une teneur en carbone d'au plus 50 ppm et plus particulièrement d'au plus 10 ppm.

Enfin, le pH des silices selon l'invention mesuré selon la norme NFT 45-007 est généralement au plus de 7. Il est plus particulièrement compris entre 5,5 et 7 et notamment 6 et 7.

Les caractéristiques ci-dessus permettent d'avoir une silice qui soit compatible au moins avec le zinc. Cette compatibilité mesurée selon le test donné ci-dessous, est d'au moins 50%, plus particulièrement d'au moins 80 % et préférentiellement d'au moins 90 %. Suivant les cas, la silice de l'invention peut être compatible en plus avec les fluorures, les phosphates et leurs dérivés.

Outre les caractéristiques de chimie de surface qui viennent d'être décrites ci-dessous et qui conditionnent les compatibilités, les silices de l'invention présentent aussi des caractéristiques physiques qui les rendent parfaitement adaptées à leur application en dentifrice. Ces caractéristiques de type structure vont être décrités ci-dessous.

Généralement la surface BET des silices de l'invention est comprise entre 40 et 600 $m^2/g$ plus particuliérement entre 40 et 350 $m^2/g$. Leur surface CTAB varie habituellement entre 40 et 400 $m^2/g$, plus particulièrement entre 40 et 200 $m^2/g$.

La surface BET est déterminée selon la méthode de BRUNAUER-EMMET-TELLER décrite dans the Journal of the American Chemical Society vol. 60, page 309, February 1938 et selon la norme NF X11-622-(3.3).

La surface CTAB est la surface externe déterminée selon la norme ASTM D3765 mais en pratiquant l'adsorption de bromure d'hexadécyltriméthyl d'ammonium (CTAB) à pH 9 et en prenant comme aire projetée moyenne de la molécule de CTAB 35 $A^{*2}$.

Les silices de l'invention peuvent bien entendu correspondre aux trois type habituellement distingués dans le domaine du dentifrice.

Ainsi, les silices de l'invention peuvent être du type abrasif. Elles présentent alors une surface BET comprise entre 40 et 300 m2/g. Dans ce cas, la surface CTAB est comprise entre 40 et 100 m2/g.

Les silices de l'invention peuvent aussi être du type épaississant. Elles ont alors une surface BET comprise entre 120 et 450 m2/g plus particulièrement 120 et 200 m2/g. Elles pourront présenter alors une surface CTAB entre 120 et 400 m2/g, plus particulièrement entre 120 et 200 $m^2/g$.

Enfin, selon un troisième type, les silices de l'invention peuvent être bifonctionnelles. Elles possèdent ici une surface BET comprise entre 80 et 200 m2/g. La surface CTAB est alors comprise entre 80 et 200 m2/g.

Les silices de l'invention peuvent aussi présenter une prise d'huile comprise entre 80 et 500 cm3/100 g déterminée selon la norme NFT 30-022 (mars 1953) en mettant en oeuvre le phtalate de dibutyle.

Plus précisément, cette prise d'huile sera comprise entre 100 et 140 $cm^3/100$ g pour les silices abrasives, 200 et 400 pour les silices épaississantes et 100 et 300 pour les bifonctionnelles.

Par ailleurs, toujours en vue de l'application en dentifrice, les silices sont préférentiellement une taille de particules comprise entre 1 et 10 $\mu$m. Cette taille moyenne de particules est mesurée par Counter-Coulter.

La densité apparente variera généralement entre 0,01 et 0,3.

Selon un mode de réalisation particulier de l'invention, les silices sont des silices de précipitation.

Enfin, les silices de l'invention présentent un indice de réfraction compris entre 1,440 et 1,465.

Le procédé de préparation des silices de l'invention va maintenant être décrit plus particulièrement.

Comme indiqué plus haut, ce procédé est du type comprenant la réaction d'un silicate avec un acide ce qui donne lieu à la formation d'une suspension ou d'un gel de silice.

Il est à noter que l'on peut utiliser tout mode opératoire connu pour arriver à cette suspension ou ce gel (Addition d'acide sur un pied de cuve de silicate, addition simultanée totale ou partielle d'acide et de silicate sur un pied de cuve d'eau ou de suspension de silicate, etc...), le choix se faisant essentiellement en fonction des caractéristiques physiques de la silice que l'on désire obtenir. On notera qu'il peut être avantageux d'amener le pH de la suspension ou du gel obtenue à une valeur d'au plus 6 et plus particulièrement comprise entre 4 et 6.

On procède alors à la séparation de la silice du milieu réactionnel selon tout moyen connu, filtre sous vide ou filtre presse par exemple.

On recueille ainsi un gâteau de silice.

Selon la caractéristique principale du procédé on procède alors à un premier lavage du gâteau.

Le premier lavage se fait à l'eau généralement de l'eau déionisée.

Le procédé comporte ensuite un second lavage ou traitement avec une solution acide.

Ce second lavage ou traitement a pour but d'obtenir à la fin de la préparation une silice ayant un pH d'au plus 7 et plus particulièrement compris entre 5,5 et 7 et notamment 6 et 7 ainsi qu'un PZC compris entre 3 et 6,5.

Cette solution acide peut être par exemple une solution d'un acide minéral tel que l'acide nitrique.

Par ailleurs selon un autre mode de réalisation, cette solution acide peut aussi être une solution d'un acide organique notamment un acide complexant. Cet acide pourra être choisi dans le groupe des acides carboxyliques, dicarboxyliques, hydroxycarboxyliques et amino-carboxyliques.

On peut citer comme exemple de tels acides l'acide acétique et pour les acides complexants, l'acide tartrique, l'acide maléique, l'acide glycérique, l'acide gluconique, l'acide citrique, l'acide acétique.

Ce second lavage ou traitement peut se faire par passage de la solution acide sur le gâteau ou introduction de celle-ci dans la suspension obtenue après délitage du gâteau. Ce lavage ou traitement acide est réalisé dans des conditions telles que pour obtenir une silice ayant le pH final indiqué plus haut, le pH de la suspension ou du milieu avant sechage doit se situer entre 4 et 6 et plus particulièrement 5 et 6.

Il peut être avantageux surtout dans le cas de l'emploi d'une solution d'un acide minéral de procéder à un ultime lavage par de l'eau déionisée.

Selon une autre variante particulière, après la réaction acide silicate et juste avant la séparation de la silice, on réalise un mûrissement de la suspension ou du gel. Ce mûrissement se fait généralement à un pH d'au plus 6 et compris entre 4 et 6 par exemple. Il est aussi possible de réaliser un mûrissement en cours de réaction à un pH entre 6 et 8. Ces mûrissements se font de préférence à chaud par exemple à une température comprise entre 80 et 100°C, et sur une durée qui peut varier entre quinze minutes et deux heures.

Une fois le gâteau de silice lavé ou traité selon les modes opératoires décrits plus haut celui-ci ou, s'il est délité, la suspension de délitage, est séché selon tout moyen connu. Le séchage peut se faire notamment par atomisation. Le produit séché sera broyé si nécessaire pour obtenir la granulométrie désirée.

L'invention concerne aussi des compositions dentrifrice contenant les silices du type décrit ci-dessus.

La quantité de silice selon l'invention utilisée dans les compositions dentifrices peut varier dans de larges limites, elle est habituellement comprise entre 5 et 35% en poids.

Les silices de l'invention s'appliquent particulièrement bien aux compositions dentifrices contenant au moins en élément choisi dans le groupe comprenant les fluorures, les phosphates, le zinc.

En ce qui concerne les composés fluorés, leur quantité correspond de préférence à une concentration en fluor dans la composition comprise entre 0,01 et 1 % en poids et plus particulièrement 0,1% à 0,5 %. Les composés fluorés sont en particulier les sels de l'acide monofluorophosphorique et particulièrement ceux de sodium, potassium, lithium, calcium, aluminium et ammonium, le mono et le difluorophosphate ainsi que des fluorures variés contenant le fluor sous forme d'ion lié particulièrement les fluorures alcalins comme ceux de sodium, lithium, potassium, le fluorure d'ammonium, le flurorue stanneux, le fluorure de manganèse, le fluorure de zirconium, le fluorure d'aluminium ainsi que des produits d'addition de ces fluorures entre eux ou avec d'autres fluorures, tels que les fluorures de potassium ou de sodium ou de manganèse.

D'autres fluorures sont également utilisables pour la présente invention comme, par exemple, le fluorure de zinc, le fluorure de germanium, le fluorure de palladium, le fluorure de titane, les fluozirconates alcalins par exemple de sodium ou de potassium, le fluozirconate stanneux, le fluoborate ou les fluosulfates de sodium, de potassium.

Les composés fluorés organiques peuvent également être utilisés, de préférence ceux connus comme les produits d'addition d'amines ou d'amino-acides à longue chaîne avec le fluorure d'hydrogène, le fluorure de cétylamine, le dihydrofluorure de bis-(hydroxyéthyl)aminopropyl N-hydroxyéthyl octadécylamine, le fluorure d'octadécylamine et de dihydrofluorure de N, N', N'tri-(polyoxyéthylène) N-hexadécylpropylènodiamine.

En ce qui concerne le zinc, celui-ci est présent notamment sous forme citrate ou sulfate.

Pour les éléments utilisables comme agents anti-plaques du type polyphosphates ou polyphosphonates, guanidines, bis-biguanides on peut mentionner ceux indiqués dans les brevets US 3.934.002 ou 4.110.083 dont l'enseignement est incorporé ici.

Les compositions dentifrices peuvent contenir en outre un liant.

Les principaux liants utilisés sont notamment choisis parmi :

- les dérivés cellulosiques : méthylcellulose, hydroxyéthylcellulose, carboxyméthylcellulose sodique,
- les mucilages : carraghénates, alginates, agar-agar et géloses,
- les gommes : gommes arabique et adragante, gomme xanthane, gomme Karaya,
- les polymères carboxyvinyliques et acryliques,

5

- les résines de polyoxyéthylène.

Outre les silices de l'invention, les compositions dentifrices peuvent contenir aussi un ou plusieurs autres agents abrasifs polissants choisis notamment parmi :
- le carbonate de calcium précité,
- le carbonate de magnésium,
- les phosphates de calcium, di-et tricalciques,
- le métaphosphate de sodium insoluble,
- le pyrophosphate de calcium,
- l'oxyde de titane (agent de blanchiment),
- les silicates,
- les alumines et silico-aluminates,
- les oxydes de zinc et d'étain,
- le talc,
- le kaolin.

Les compositions dentifrices peuvent aussi comprendre des détergents, des humectants, des agents aromatisants, édulcorants et des colorants et des conservateurs.

Les principaux détergents utilisés sont notamment choisis parmi :
- le laurylsulfate de sodium,
- le lauryléthersulfate et le laurylsulfoacétate de sodium,
- le dioctylsulfosuccinate de sodium,
- le laurylsarcosinate de sodium,
- le ricinoléate de sodium,
- les monoglycérides sulfatés.

Les principaux agents humectants utilisés sont notamment choisis parmi les polyalcools comme :
- le glycérol,
- le sorbitol, généralement en solution à 70% dans l'eau,
- le propylène glycol.

Les principaux agents aromatisants (parfum) sont notamment choisis parmi : les essences d'anis, de badiane, de menthe, de baie de genièvre, de cannelle, de girofle et de rose.

La principauyx agents édulcorants sont notamment choisis parmi les imides orthosulfobenzoïques et les cyclamates.

Les principaux colorants utilisés sont notamment choisis selon la couleur désirée parmi :
- coloration rouge et rose : amaranthe, azorubine, cachou, coccine nouvelle (PONCEAU 4R), cochenille, érythrosine,
- coloration verte : chlorophylle et chlorophylline,
- coloration jaune : jaune soleil (Orange S) et jaune de quinoléine.

Les principaux conservateurs les plus utilisés sont : les parahydroxybenzoates, le formol et les produits qui en dégagent, l'héxétidine, les ammoniums quaternaires, l'hexachlorophène, le bromophène et l'hexamédine.

Enfin, les compositions dentifrices contiennent des agents thérapeutiques dont les principaux sont notamment choisis parmio :
- les antiseptiques et les antibiotiques,
- les enzymes,
- les oligo-éléments et les composés fluorés qui ont été décrits ci-dessus.

Des exemples concrets mais non limitatifs vont maintenant être donnés. Auparavant les tests pour la mesure de la compatibilité de la silice avec différents éléments vont être décrits.

Mesure de la compatibilité avec les fluorures

4 g de silice sont dispersés dans 16 g de solution à 0,396 de fluorure de sodium (NaF). La suspension est agitée pendant 24 heures à 37°C. Après centrifugation de la suspension à 20000 t/mn pendant 30 mn, le surnageant est filtré sur filtre Millipore 0,2 $\mu$m. La solution ainsi obtenue, constitue la solution de l'essai.

Une solution de référence est constituée en utilisant le même protocole mais en absence de silice.

La compatibilité avec les fluorures est déterminée par le % de fluorure libre mesuré par électrode sélective à fluorure (Orion). Elle est déterminée par la relation ci-dessous.

$$\% \text{ Compatibilité} = \frac{\text{Concentration en F de l'essai (ppm)}}{\text{Concentration en F de la référence (ppm)}} \times 100$$

### Mesure de la compatibilité avec le zinc

4 g de silice sont dispersés dans 100 ml de solution à 0,06% de $ZnSO_4$, $7H_2O$. On obtient une suspension dont le pH est stabilisé à 7 pendant 15 minutes par ajout de NaOH ou $H_2SO_4$. La suspension est agitée ensuite pendant 24 heures à 37°C puis est centrifugée à 20000 t/mn pendant 30 mn.

Le surnageant filtré sur filtre Millipore 0,2 $\mu$m, constitue la solution de l'essai.

Une solution de référence est constituée en suivant le même protocole mais en absence de silice.

La concentration en zinc libre des deux solutions est déterminée par absorption, atomique (214 nm).

La compatibilité est déterminée par la relation ci-dessous :

$$\% \text{ Compatiblité} = \frac{\text{Concentration en Zn de l'essai (ppm)}}{\text{Concentration en Zn de la référence (ppm)}} \times 100$$

### Mesure de la compatibilité avec les pyrophosphates de sodium et de potassium

4 g de silice sont dispersés dans 16g de suspension à 1,5% de pyrophosphate de sodium ou de potassium. La suspension est agitée pendant 24 heures à 37°C puis est centrifugée à 20000 t/mn pendant 30 mn.

Le surnageant est filtré sur filtre Millipore 0,2 $\mu$m.0,2g de solution diluée dans 100 ml d'eau dans une fiole jaugée, constitue la solution de l'essai.

Une solution de référence est constituée en suivant le même protocole mais en absence de silice.

La concentration en ion pyrophosphate ($P_2O_7^-$) libre des deux solutions est déterminée par chromatographie ionique (système DIONEX 2000i) équipé d'un intégrateur.

La compatibilité est déterminée par le rapport des aires des pics obtenus sur les chromatogrammes et correspondant au temps de rétention du pyrophosphate, de l'essai et de la référence.

$$\% \text{ Compatibilité} = 100 \times \frac{\text{aire du pic de l'essai}}{\text{aire du pic de la référence}}$$

### EXEMPLE 1

Dans un réacteur équipé d'un système de régulation de température et de pH et d'un système d'agitation par turbine, on introduit 6 l d'eau déionisée.

Après la mise en marche de l'agitation (300 t/mn), le pied de cuve ainsi constitué est chauffé à 85 °C.

Lorsque la température est atteinte, on procède à l'addition simultanée de 8,5 l de silicate de sodium de concentration en silice de 120 g/l, de rapport $SiO_2/Na_2O$ égal à 3,5 et de débit 0,34 l/mn et de 13,5 l d'acide sulfurique de concentration 80 g/l. Le débit d'acide est ajusté de manière à maintenir le pH du milieu à une valeur constante de 8,0.

Après 40 mn d'addition, on laisse mûrir le mélange pendant 10 mn à ce pH et à cette température.

On arrête l'addition de silicate et on continue l'addition d'acide jusqu'à stabiliser le pH du mélange réactionnel à 4.

On réalise par la suite, un mûrissement de 15 mn à ce pH et à 85°C.

Le mélange est ensuite filtré et le gâteau humide est lavé à l'eau déionisée.

Le gâteau est ensuite dispersé dans de l'eau déionisée pour former une suspension homogène de concentration en silice de 50 g/l. On ajuste le pH de cette suspension à 5,8 par ajout d'acide nitrique et on laisse le mélange se stabiliser à ce pH pendant 15 mn.

La suspension est filtrée.

Le produit est ensuite séché par atomisation et broyé sur un broyeur de type forplex pour obtenir une granulométrie de 9 microns.

Les caractéristiques physico-chimiques de la silice ainsi obtenue sont les suivantes :

| | |
|---|---|
| Surface BET | 90 m²/g |
| Surface CTAB | 60 m²/g |
| Prise d'huile | 105 cm³/100g |
| pH | 6,8 |
| nombre de OH/nm2 | 8. |

Les analyses chimiques de la silice sont regroupées dans le tableau ci-dessous.

| ions | Al | Fe | Ca | C |
|------|-----|-----|-----|-----|
| ppm | 350 | 110 | 300 | 10 |

Le PZC de la silice est de 4,5.

Dans le tableau ci-dessous sont regroupées les différentes compatibilités de la silice ainsi obtenue avec les ingrédients d'une formulation dentifrice et mesurées à l'aide des différents tests décrits par ailleurs:

| Ingrédients | Fluorure NaF | Pyrophosphate Na/K | Zinc ZnSO₄ |
|-------------|--------------|--------------------|-----------|
| % Compatible | 90 | 98 | 90 |

EXEMPLE COMPARATIF 2

A titre comparatif on donne ci-dessous des mesures de compatibilité avec des silices commerciales généralement utilisées dans des formulations dentifrices, ainsi que leurs caractéristiques physico-chimiques.

| Silice Marque Fabricant | Surface m²/g | | Nombre OH/nm² | Compatibilité | | |
|-------------------------|------|------|---------------|------|------|---------------|
| | CTAB | BET | | Zn | F | Pyrophosphate |
| Zeodent 113 Hubert | 50 | 100 | 30 | 0 | 95 | 95 |
| Tixosil 53 Rhône-Poulenc | 50 | 250 | 30 | 0 | 60 | 90 |
| Z 119 Rhône-Poulenc | 50 | 60 | 25 | 20 | 95 | 95 |

On notera que pour les silices de ce tableau le PZC est inférieur à 3.

EXEMPLE 3

Cet exemple concerne la formulation d'un dentifrice opaque du type pâte :

La formule est la suivante :

| Glycérine | 22,00 |
|---|---|
| CMC 7mFD | 1.00 |
| Saccharinate de sodium | 0,20 |
| Monofluorophosphate de sodium | 0,76 |
| Fluoure de sodium | 0,10 |
| Lauryl sulfate de sodium (30% aqueux) | 4,67 |
| Benzoate de sodium | 0,10 |
| Arôme | 0,90 |
| Dioxyde de titane | 1,00 |
| Silice de l'exemple 1 | 31,50 |
| $ZnSO_4$, 7 $H_2O$ | 0,48 |
| Eau distillée | 37,29. |

L'expertise rhéologique et visuelle de la pâte dentifrice obtenue montre que les propriétés usuelles du dentifrice sont bonnes.

**Revendications**

1.  Silice caractérisée en ce qu'elle présente une chimie de surface telle que le nombre de OH exprimé en OH/nm2 soit d'au plus 15 et que son point de charge nulle (PZC) soit compris entre 3 et 6,5 et une compatibilité avec le zinc d'au moins 50 %.

2.  Silice selon la revendication 1, caractérisée en ce que le nombre de OH est d'au plus 12 notamment compris entre 3 et 12.

3.  Silice selon l'une des revendications précédentes, caractérisée en ce qu'elle présente une teneur en aluminium d'au plus 500 ppm.

4.  Silice selon l'une des revendications précédentes, caractérisée en ce qu'elle présente une teneur en fer d'au plus 200 ppm.

5.  Silice selon l'une des revendications précédentes, caractérisée en ce qu'elle présente une teneur en calcium d'au plus 500 ppm, en particulier d'au plus 300 ppm.

6.  Silice selon l'une des revendications précédentes, caractérisée en ce qu'elle présente une teneur en carbone d'au plus 50 ppm et plus particulièrement d'au plus 10 ppm.

7.  Silice selon l'une des revendications précédentes, caractérisée en ce qu'elle présente un pH d'au plus 7 et plus particulièrement comprises entre 5,5 et 7.

8.  Silice selon l'une des revendications précédentes, caractérisée en ce qu'elle présente une surface BET comprise entre 40 et 600 m2/g.

9.  Silice selon l'une des revendications précédentes, caractérisée en ce qu'elle présente une surface CTAB comprise entre 40 et 400 m2/g.

10. Silice selon l'une des revendications précédentes, du type abrasif caractérisé en ce qu'elle présente une surface BET comprise entre 40 et 300 m2/g.

11. Silice selon la revendication 10, caractérisée en ce qu'elle présente une surface CTAB comprise entre 40 et 100 m2/g.

12. Silice selon l'une quelconque des revendications 1 à 9, du type épaississant caractérisée en ce qu'elle présente une surface BET comprise entre 120 et 450 m2/g plus particulièrement 120 et 200 m2/g.

13. Silice selon la revendication 12, caractérisée en ce qu'elle présente une surface CTAB comprise entre 120 et 400 m2/g.

**14.** Silice selon l'une quelconque des revendications 1 à 9, du type bifonctionnelle, caractérisée en ce qu'elle présente une surface BET comprise entre 80 et 200 m2/g.

**15.** Silice selon la revendication 14, caractérisée en ce qu'elle présente une surface CTAB comprise entre 80 et 200 m2/g.

**16.** Silice selon l'une des revendications précédentes, caractérisée en ce qu'elle présente une prise d'huile comprise entre 80 et 500 cm3/100g.

**17.** Silice selon l'une des revendications précédentes, caractérisée en ce qu'elle présente une taille moyenne de particules comprise entre 1 et 10 $\mu$m.

**18.** Silice selon l'une des revendications précédentes, caractérisée en ce qu'il s'agit d'une silice de précipitation.

**19.** Composition dentifrice, caractérisée en ce qu'elle contient une silice selon l'une des revendications 1 à 18.

**20.** Composition dentifrice selon la revendication 19 caractérisée en ce qu'elle contient au moins un élément choisi dans le groupe comprenant le fluor, les phosphates, le zinc.

**Claims**

**1.** A silica, characterised in that it has a surface chemistry such that the number of OH expressed in $OH/nm^2$ is at the most 15 and that its point zero charge (PZC) is between 3 and 6.5 and its compatibility with zinc is at least 50%.

**2.** A silica according to Claim 1, characterised in that the number of OH is at the most 12, in particular between 3 and 12.

**3.** A silica according to one of the preceding claims, characterised in that it has an aluminium content of at the most 500 ppm.

**4.** A silica according to one of the preceding claims, characterised in that it has an iron content of at the most 200 ppm.

**5.** A silica according to one of the preceding claims, characterised in that it has a calcium content of at the most 500 ppm, in particular of at the most 300 ppm.

**6.** A silica according to one of the preceding claims, characterised in that it has a carbon content of at the most 50 ppm, and more particularly of at the most 10 ppm.

**7.** A silica according to one of the preceding claims, characterised in that it has a pH of at the most 7, and more particularly of between 5.5 and 7.

**8.** A silica according to one of the preceding claims, characterised in that it has a BET surface of between 40 and 600 $m^2/g$.

**9.** A silica according to one of the preceding claims, characterised in that it has a CTAB surface of between 40 and 400 $m^2/g$.

**10.** A silica according to one of the preceding claims, of the abrasive type, characterised in that it has a BET surface of between 40 and 300 $m^2/g$.

**11.** A silica according to Claim 10, characterised in that it has a CTAB surface of between 40 and 100 $m^2/g$.

**12.** A silica according to any one of Claims 1 to 9, of the thickening type, characterised in that it has a BET surface of between 120 and 450 $m^2/g$, more particularly of between 120 and 200 $m^2/g$.

13. A silica according to Claim 12, characterised in that it has a CTAB surface of between 120 and 400 $m^2$/g.

14. A silica according to any one of Claims 1 to 9, of the bifunctional type, characterised in that it has a BET surface of between 80 and 200 $m^2$/g.

15. A silica according to Claim 14, characterised in that it has a CTAB surface of between 80 and 200 $m^2$/g.

16. A silica according to one of the preceding claims, characterised in that it has an oil sample of between 80 and 500 $cm^3$/100 g.

17. A silica according to one of the preceding claims, characterised in that it has a mean particle size of between 1 and 10 $\mu$m.

18. A silica according to one of the preceding claims, characterised in that it is a precipitation silica.

19. A dentifrice composition, characterised in that it contains a silica according to one of Claims 1 to 18.

20. A dentifrice composition according to Claim 19, characterised in that it contains at least one element selected from the group including fluorine, phosphates, zinc.

**Patentansprüche**

1. Kieselsäure, dadurch gekennzeichnet, daß sie eine Oberflächenchemie aufweist, bei der die Anzahl der OH-Gruppen, ausgedrückt in OH/$nm^2$, höchstens 15 ist und ihr isoelektrischer Punkt (PZC) zwischen 3 und 6,5 liegt und sie eine Kompatibilität mit Zink von mindestens 50 % hat.

2. Kieselsäure nach Anspruch 1, dadurch gekennzeichnet, daß die Anzahl der OH-Gruppen höchstens 12 ist, insbesondere zwischen 3 und 12.

3. Kieselsäure nach einem der vorhergehenden Ansprüche, dadurch gekennzeichnet, daß sie einen Aluminiumgehalt von höchstens 500 ppm besitzt.

4. Kieselsäure nach einem der vorhergehenden Ansprüche, dadurch gekennzeichnet, daß sie einen Eisengehalt von höchstens 200 ppm besitzt.

5. Kieselsäure nach einem der vorhergehenden Ansprüche, dadurch gekennzeichnet, daß sie einen Calciumgehalt von höchstens 500 ppm, insbesondere von höchstens 300 ppm, besitzt.

6. Kieselsäure nach einem der vorhergehenden Ansprüche, dadurch gekennzeichnet, daß sie einen Kohlenstoffgehalt von höchstens 50 ppm und insbesondere von höchstens 10 ppm besitzt.

7. Kieselsäure nach einem der vorhergehenden Ansprüche, dadurch gekennzeichnet, daß sie einen pH-Wert von höchstens 7, insbesondere zwischen 5,5 und 7, besitzt.

8. Kieselsäure nach einem der vorhergehenden Ansprüche, dadurch gekennzeichnet, daß sie eine BET-Oberfläche zwischen 40 und 600 $m^2$/g besitzt.

9. Kieselsäure nach einem der vorhergehenden Ansprüche, dadurch gekennzeichnet, daß sie eine CTAB-Oberfläche zwischen 40 und 400 $m^2$/g besitzt.

10. Kieselsäure nach einem der vorhergehenden Ansprüche vom Schleifmitteltyp, dadurch gekennzeichnet, daß sie eine BET-Oberfläche zwischen 40 und 300 $m^2$/g besitzt.

11. Kieselsäure nach Anspruch 10, dadurch gekennzeichnet, daß sie eine CTAB-Oberfläche zwischen 40 und 100 $m^2$/g besitzt.

EP 0 317 378 B2

**12.** Kieselsäure nach einem der Ansprüche 1 bis 9 vom Verdickungsmitteltyp, dadurch gekennzeichnet, daß sie eine BET-Oberfläche zwischen 120 und 450 m$^2$/g, insbesondere zwischen 120 und 200 m$^2$/g, besitzt.

**13.** Kieselsäure nach Anspruch 12, dadurch gekennzeichnet, daß sie eine CTAB-Oberfläche zwischen 120 und 400 m$^2$/g besitzt.

**14.** Kieselsäure nach einem der Ansprüche 1 bis 9 vom bifunktionellen Typ, dadurch gekennzeichnet, daß sie eine BET-Oberfläche zwischen 80 und 200 m$^2$/g besitzt.

**15.** Kieselsäure nach Anspruch 14, dadurch gekennzeichnet, daß sie eine CTAB-Oberfläche zwischen 80 und 200 m$^2$/g besitzt.

**16.** Kieselsäure nach einem der vorhergehenden Ansprüche, dadurch gekennzeichnet, daß sie ein Ölaufnahmevermögen zwischen 80 und 500 cm$^3$/100 g besitzt.

**17.** Kieselsäure nach einem der vorhergehenden Ansprüche, dadurch gekennzeichnet, daß sie eine mittlere Teilchengröße zwischen 1 und 10 $\mu$m besitzt.

**18.** Kieselsäure nach einem der vorhergehenden Ansprüche, dadurch gekennzeichnet, daß gefällte Kieselsäure vorliegt.

**19.** Zahnpastazusammensetzung, dadurch gekennzeichnet, daß sie Kieselsäure nach einem der Ansprüche 1 bis 18 enthält.

**20.** Zahnpastazusammensetzung nach Anspruch 19, dadurch gekennzeichnet, daß sie wenigstens ein Element, ausgewählt aus der Gruppe, bestehend aus Fluor, Phosphaten und Zink, enthält.